(19) Europäisches Patentamt
European Patent Office
Office européen des brevets

(11) Veröffentlichungsnummer: **0 200 145**
**B1**

(12) **EUROPÄISCHE PATENTSCHRIFT**

(45) Veröffentlichungstag der Patentschrifft: **28.02.90**

(51) Int. Cl. [5]: **C 07 D 249/08**, C 07 D 233/60, C 07 B 57/00

(21) Anmeldenummer: **86105569.7**

(22) Anmeldetag: **22.04.86**

(54) **Verfahren zur Herstellung optisch aktiver Azolderivate.**

(30) Priorität: **03.05.85 DE 3515869**

(43) Veröffentlichungstag der Anmeldung:
**05.11.86 Patentblatt 86/45**

(45) Bekanntmachung des Hinweises auf die Patenterteilung:
**28.02.90 Patentblatt 90/09**

(84) Bennante Vertragsstaaten:
**AT BE CH DE FR GB IT LI NL**

(56) Entgegenhaltungen:
**EP-A-0 054 431**
**EP-A-0 114 608**
**EP-A-0 114 609**
**EP-A-0 200 146**

(73) Patentinhaber: **BAYER AG**
**D-5090 Leverkusen 1 Bayerwerk (DE)**

(72) Erfinder: **Priesnitz, Uwe, Dr.**
**Severinstrasse 58**
**D-5650 Solingen 1 (DE)**

EP 0 200 145 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBERGRAF, STOCKHOLM 1990

**Beschreibung**

Die vorliegende Erfindung betrifft ein neues Verfahren zur Herstellung von optisch aktiven Azolderivaten mit fungizider und pflanzenwachstumsregulierender Wirksamkeit.

Es ist bereits bekannt geworden, Racemate von bestimmten Azolderivaten mit Hilfe von optisch aktiven Komponenten in einzelne Enantiomere zu trennen (vgl. EP-A-0 114 608, EP-A-0 114 609, US-A-4 435 203 und EP-A-0 054 431).

Diese Verfahren sind jedoch mit einigen Nachteilen behaftet. So ist die optische Reinheit der resultierenden Verbindungen nicht immer voll befriedigend. Außerdem lassen sich die beiden in Frage kommenden Enantiomeren in vielen Fällen nicht gleichermaßen gut herstellen, da von den benötigten optisch aktiven Hilfsreagentien nicht immer beide Antipoden ausreichend rein verfügbar sind. Im übrigen werden die optisch aktiven Azolderivate häufig in einer für praktische Zwecke zu niedrigen Ausbeute erhalten.

Es wurde nun gefunden, daß sich optisch aktive Azolderivate der Formel

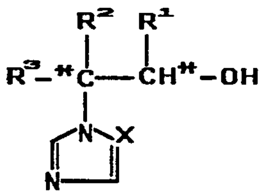

(I)

$R^1$ für geradkettiges oder verzweigtes Alkyl mit 1 bis 4 Kohlenstoffatomen, für gegebenenfalls einfach oder mehrfach durch Alkyl mit 1 bis 2 Kohlenstoffatomen substituiertes Cycloalkyl mit 3 bis 7 Ring-Kohlenstoffatomen sowie für Phenyl oder Naphthyl, wobei jeder der beiden zuvor genannten Reste einfach oder mehrfach, gleichartig oder verschieden substituiert sein kann durch Halogen, geradkettiges oder verzweigtes Alkyl mit 1 bis 4 Kohlenstoffatomen sowie Halogenalkyl mit 1 oder 2 Kohlenstoffatomen und 1 bis 5 gleichen oder verschiedenen Halogenatomen, wie Fluor- und Chloratomen, und

$R^1$ weiterhin für einen 5- oder 6-gliedrigen, gegebenenfalls benzannellierten Heterocyclus mit 1 bis 3 gleichen oder verschiedenen Heteroatomen, wie Sauerstoff, Stickstoff und Schwefel steht, wobei der Heterocyclus einfach oder mehrfach, gleichartig oder verschieden substituiert sein kann durch Halogen. Alkyl mit 1 bis 3 Kohlenstoffatomen oder Halogenalkyl mit 1 oder 2 Kohlenstoffatomen und 1 bis 5 gleichen oder verschiedenen Halogenatomen, wie Fluor- oder Chloratomen, und

$R^1$ außerdem für die Reste der Formeln

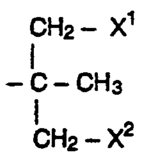

oder

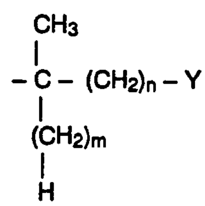

steht, wobei

$X^1$ für Wasserstoff oder Halogen steht,

$X^2$ für Halogen steht,

Y für geradkettiges oder verzweigtes Alkyl mit 1 bis 6 Kohlenstoffatomen, Alkoxy mit 1 bis 6 Kohlenstoffatomen, Alkylthio mit 1 bis 6 Kohlenstoffatomen, Halogenalkoxy mit 1 bis 6 Kohlenstoffatomen und 1 bis 9 gleichen oder verschiedenen Halogenatomen, Halogenalkylthio mit 1 bis 6 Kohlenstoffatomen und 1 bis 9 gleichen oder verschiedenen Halogenatomen, Alkenyl mit 2 bis 6 Kohlenstoffatomen, geradkettiges oder verzweigtes Alkoxycarbonyl mit 1 bis 4 Kohlenstoffatomen im Alkoxyteil, Cyano sowie für Phenyl, Phenyloxy, Phenylthio, Phenylalkoxy mit 1 bis 4 Kohlenstoffatomen in der Alkoxygruppe und Phenylalkylthio mit 1 bis 4 Kohlenstoffatomen in der Alkylthiogruppe steht, wobei jeder der zuvor genannten Phenyl-Reste einfach oder mehrfach, gleichartig oder verschieden substituiert sein kann durch Halogen, Alkyl mit 1 bis 4 Kohlenstoffatomen, Alkoxy mit 1 bis 2 Kohlenstoffatomen, Alkylthio mit 1 oder Z Kohlenstoffatomen, Halogenalkyl mit 1 oder 2 Kohlenstoffatomen und 1 bis 5 gleichen oder verschiedenen Halogenatomen, wie Fluor- und Chloratomen, Halogenalkoxy mit 1 oder 2 Kohlenstoffatomen und 1 bis 5 gleichen oder verschiedenen Halogenatomen, wie Fluor- und Chloratomen, Halogenalkylthio mit 1 oder 2 Kohlenstoffatomen und 1 bis 5 gleichen oder verschiedenen Halogenatomen, wie Fluor- und Chloratomen, Cycloalkyl mit 3 bis 7 Kohlenstoffatomen, Dialkylamino mit 1 bis 4 Kohlenstoffatomen in jedem Alkylteil, Cyano, Nitro sowie geradkettiges oder verzweigtes Alkoxycarbonyl mit 1 bis 4 Kohlenstoffatomen im Alkoxyteil, und

m für eine Zahl 0 oder 1 steht,

n für eine Zahl 0, 1 oder 2 steht,

$R^2$ für Wasserstoff oder Methyl steht,

$R^3$ für Alkyl mit 1 bis 6 Kohlenstoffatomen, Alkoxy mit 1 bis 6 Kohlenstoffatomen, Cycloalkylalkyl mit 3 bis 8 Kohlenstoffatomen in der Cycloalkylgruppe und 1 bis 4 Kohlenstoffatomen im Alkylteil, gegebenenfalls ein- oder merfach, gleichartig oder verschieden durch Alkyl mit 1 bis 4 Kohlenstoffatomen, Halogen, Phenyl und/oder Nitro substituiertes Aryl mit 6 bis 10 Kohlenstoffatomen, gegebenenfalls ein- oder mehrfach, gleichartig oder verschieden durch Alkyl mit 1 bis 4 Kohlenstoffatomen, Halogen, Phenyl und/oder Nitro substituiertes Aralkyl mit 6 bis 10 Kohlenstoffatomen im Arylteil und 1 bis 4 Kohlenstoffatomen im Alkylteil, für gegebenenfalls ein- oder mehrfach, gleichartig oder verschieden durch Alkyl mit 1 bis 4 Kohlenstoffatomen, Halogen, Phenyl und/oder Nitro substituiertes Aroxy mit 6 bis 10 Kohlenstoffatomen oder für gegebenenfalls ein- oder mehrfach, gleichartig oder verschieden durch Alkyl mit 1 bis 4 Kohlenstoffatomen, Halogen, Phenyl und/oder Nitro substituiertes Aroxyalkyl mit 6 bis 10 Kohlenstoffatomen im Aroxyteil und 1 bis 4 Kohlenstoffatomen im Alkylteil steht, oder

$R^2$ und $R^3$ auch gemeinsam für die Gruppe

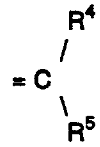 stehen, in welcher

$R^4$ für Wasserstoff, Alkyl mit 1 bis 4 Kohlenstoffatomen, Cycloalkyl mit 3 bis 8 Kohlenstoffatomen oder Cycloalkenyl mit 5 bis 8 Kohlenstoffatomen steht und

$R^5$ für Alkyl mit 1 bis 4 Kohlenstoffatomen, Cycloalkyl mit 3 bis 8 Kohlenstoffatomen, Cycloalkenyl mit 5 bis 8 Kohlenstoffatomen oder für gegebenenfalls ein- oder mehrfach, gleichartig oder verschieden durch Alkyl mit 1 bis 4 Kohlenstoffatomen und/oder Halogen substituiertes Aryl mit 6 bis 10 Kohlenstoffatomen steht oder

$R^4$ und $R^5$ gemeinsam mit dem Kohlenstoffatom, an das sie gebunden sind, für Cycloalkyl mit 5 bis 8 Kohlenstoffatomen oder für Cycloalkenyl mit 5 bis 8 Kohlenstoffatomen stehen und

X für Stickstoff oder eine CH-Gruppe steht,

dadurch herstellen lassen, daß man in einer ersten Stufe Racemate von Azolderivaten der Formel

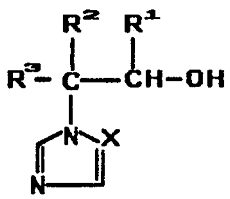

(Ia)

in welcher

$R^1$, $R^2$, $R^3$ und X die oben angegebene Bedeutung haben, mit optisch aktiven Permethrinsäurehalogeniden der Formel

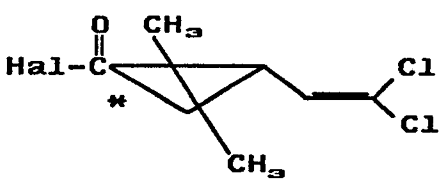

(II)

in welcher

Hal für Chlor oder Brom steht, gegebenenfalls in Gegenwart eines Säurebindemittels und gegebenenfalls in Gegenwart eines Verdünnungsmittels umsetzt, dann in einer zweiten Stufe die so erhaltenen diastereomeren Ester der Formel

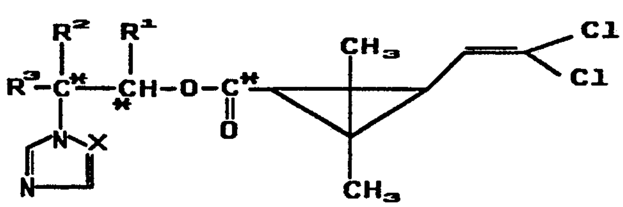

(III)

(Diastereomeren-Gemisch)

in welcher

$R^1$, $R^2$, $R^3$ und X die oben angegebene Bedeutung haben,

aufgrund ihrer unterschiedlichen physikalischen Eigenschaften trennt und danach in einer dritten Stufe das jeweilige Azolderivat der Formel (I) aus dem entsprechenden Ester mit Hilfe von Basen in Gegenwart eines

Verdünnungsmittels in Freiheit setzt.

In den oben angegebenen Formeln sind die asymmetrisch substituierten Kohlenstoffatome immer dann durch ein (*) gekennzeichnet, wenn es sich um optisch aktive Verbindungen handelt.

Es ist als äußerst überraschend anzusehen, daß sich nach dem erfindungsgemäßen Verfahren zahlreiche optisch aktive Azolderivate in sehr hoher Ausbeute und ausgezeichneter optischer Reinheit herstellen lassen, denn aufgrund des bekannten Standes der Technik konnte eine generelle Anwendung dieser Trennmethode nicht erwartet werden.

Das erfindungsgemäße Verfahren besitzt eine Reihe von Vorteilen. So sind die einzelnen Enantiomeren der als Ausgangsstoffe benötigten Permethrinsäurehalogenide auch im technischen Maßstab in extrem hoher optischer Reinheit zugänglich. Es lassen sich daher durch Einsatz des einen oder des anderen Enantiomeren gezielt Diastereomeren-Gemische von Estern der Formel (III) herstellen, aus denen der jeweils gewünschte Ester ohne Schwierigkeiten abtrennbar ist. Besonders günstig ist dabei, daß identische Trennmethoden zur Isolierung komplementärer Ester angewandt werden können. Im allgemeinen genügt eine fraktionierte Kristallisation, um den gewünschten diastereomeren Ester zu separieren. Im übrigen sind die einzusetzenden Enantiomeren der Permethrinsäurehalogenide der Formel (II) vollsynthetisch Verfahrens besteht schließlich darin, daß zahlreiche optisch aktive Azolderivate in hoher Ausbeute und ausgezeichneter optischer Reinheit zugänglich sind.

Verwendet man racemisches 1-(4-Chlorphenoxy)-1-(1,2,4-triazol-1-yl)-2-hydroxy-3,3-dimethyl-butan und 1R-trans-Permethrinsäurechlorid als Ausgangsstoffe (1. Stufe) und ein Gemisch aus Kaliumhydroxid, Wasser und Methanol zur Esterverseifung (3. Stufe), so kann der Reaktionsablauf des erfindungsgemäßen Verfahrens durch das folgende Formelschema wiedergegeben werden:

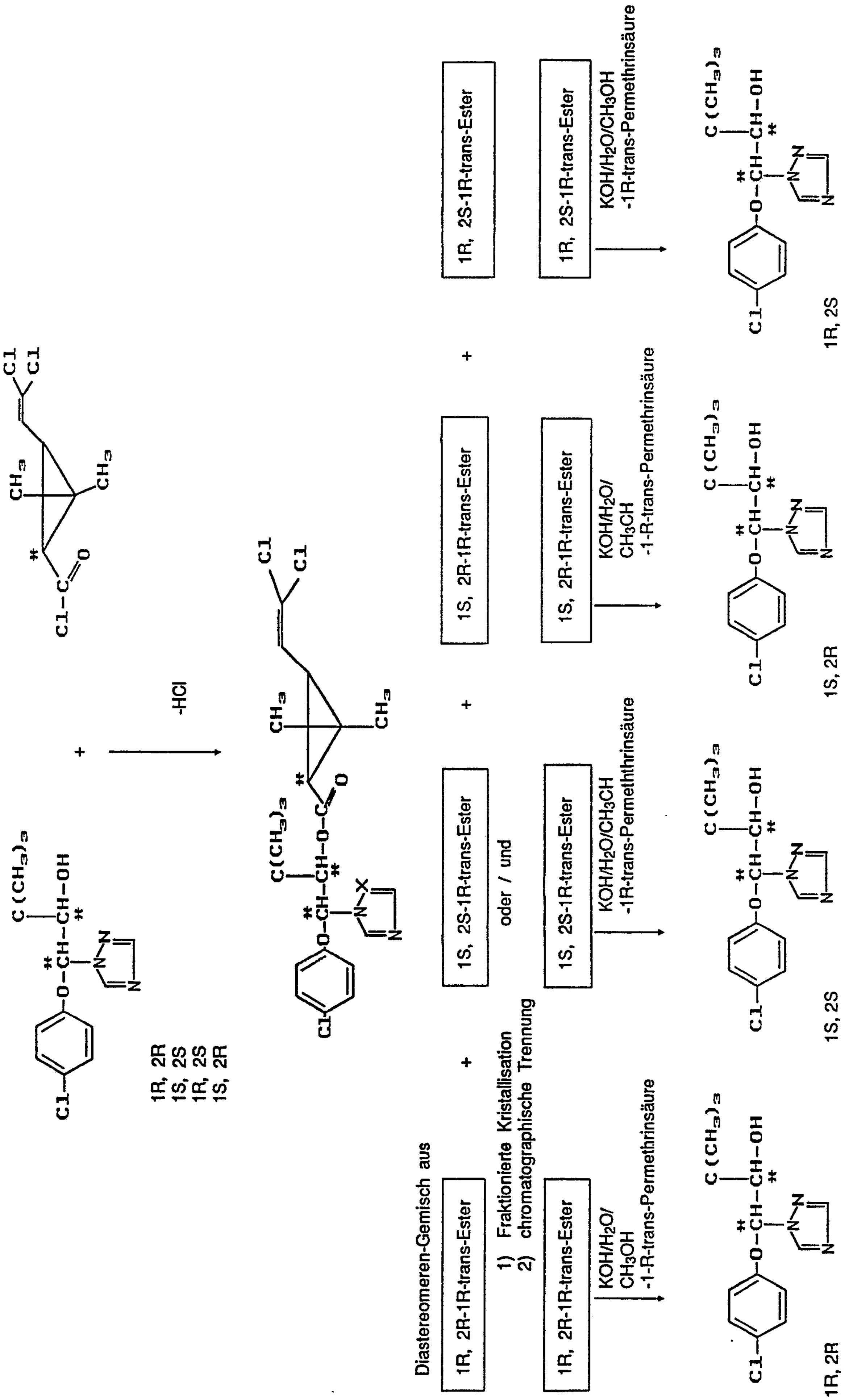
1R, 2R
1S, 2S
1R, 2S
1S, 2R
-HCl
Diastereomeren-Gemisch aus
1R, 2R-1R-trans-Ester
1S, 2S-1R-trans-Ester
1S, 2R-1R-trans-Ester
1R, 2S-1R-trans-Ester
1) Fraktionierte Kristallisation
2) chromatographische Trennung
oder / und
1R, 2R-1R-trans-Ester
KOH/H2O/CH3OH -1-R-trans-Permethrinsäure
1R, 2R
1S, 2S-1R-trans-Ester
KOH/H2O/CH3CH -1R-trans-Permethrinsäure
1S, 2S
1S, 2R-1R-trans-Ester
KOH/H2O/CH3CH -1-R-trans-Permethrinsäure
1S, 2R
1R, 2S-1R-trans-Ester
KOH/H2O/CH3OH -1R-trans-Permethrinsäure
1R, 2S

Die bei der Durchführung des erfindungsgemäßen Verfahrens als Ausgangsstoffe benötigten Racemate von Azolderivaten sind durch die Formel (Ia) definiert.

Bevorzugt sind Racemate von Azolderivaten der Formel (Ia), in denen

$R^1$ für Methyl, Ethyl, n- oder i-Propyl, n-, i-, s- oder t-Butyl, für gegebenenfalls ein- bis dreifach gleich oder verschieden durch Methyl und/oder Ethyl subtituiertes Cyclopropyl, Cyclopentyl oder Cyclohexyl, sowie für Phenyl oder Naphthyl steht, wobei jeder der beiden zuvor genannten Reste ein- bis dreifach, gleichartig oder verschieden substituiert sein kann durch Fluor, Chlor, Brom, Iod, Methyl, Ethyl, n-Propyl, Isopropyl, Trifluormethyl und Trichlormethyl, und

$R^1$ außerdem für die Reste der Formeln

```
  CH2 - X1                      CH3
  |                             |
- C - CH3          oder       - C - (CH2)n - Y
  |                             |
  CH2-X2                        (CH2)m
                                |
                                H
```

steht, wobei

$X^1$ für Wasserstoff oder Halogen steht,

$X^2$ für Halogen steht,

Y für Methyl, Ethyl, n- oder i-Propyl, Methoxy, Ethoxy, Methylthio, Ethylthio, Trifluormethoxy, Trifluormethylthio, Vinyl, Allyl, Methoxycarbonyl, Ethoxycarbonyl, Cyano sowie für Phenyl, Phenoxy, Phenylthio, Benzyloxy oder Benzylthio steht, wobei jeder der fünf letztgenannten Reste im Phenylteil einfach bis dreifach, gleichartig oder verschieden substituiert sein kann durch Fluor, Chlor, Brom, Methyl, Ethyl, Methoxy, Methylthio, Trifluormethyl, Trifluormethoxy, Trifluormethylthio, Dimethylamino, Diethylamino, Methoxycarbonyl, Ethoxycarbonyl, Cyano oder Nitro,

m für eine Zahl 0 oder 1 steht und

n für eine Zahl 0, 1 oder 2 steht,

$R^2$ für Wasserstoff oder Methyl steht,

$R^3$ für Alkyl mit 1 bis 4 Kohlenstoffatomen, Alkoxy mit 1 bis 4 Kohlenstoffatomen, Cycloalkylalkyl mit 5 bis 7 Kohlenstoffatomen im Cycloalkylteil und 1 bis 3 Kohlenstoffatomen im Alkylteil, gegebenenfalls ein- bis dreifach, gleichartig oder verschieden durch Methyl, Ethyl, Propyl, Fluor, Chlor, Brom, Phenyl und/oder Nitro substituiertes Phenyl, für gegebenenfalls ein- bis dreifach, gleichartig oder verschieden durch Methyl, Ethyl, Propyl, Fluor, Chlor, Brom, Phenyl und/oder Nitro substituiertes Phenylalkyl mit 1 oder 2 Kohlenstoffatomen im Alkylteil, für gegebenenfalls ein- bis dreifach, gleichartig oder verschieden durch Methyl, Ethyl, Propyl, Fluor, Chlor, Brom, Phenyl und/oder Nitro substituiertes Phenoxy, oder für gegebenenfalls ein- bis dreifach, gleichartig oder verschieden durch Methyl, Ethyl, Propyl, Fluor, Chlor, Brom, Phenyl und/oder Nitro substituierts Phenoxyalkyl mit 1 oder 2 Kohlenstoffatomen im Alkylteil steht oder

```
                                              R4
                                             /
R2 und R3 gemeinsam für die Gruppe = C            stehen,
                                             \
                                              R5
```

in welcher

$R^4$ für Wasserstoff, Methyl, Ethyl, Propyl, Cyclopentyl, Cyclohexyl, Cyclopentenyl oder Cyclohexenyl steht und

$R^5$ für Methyl, Ethyl, Propyl, Cyclopentyl, Cyclohexyl, Cyclopentenyl, Cyclohexenyl oder für gegebenenfalls ein- bis dreifach, gleichartig oder verschieden durch Methyl, Ethyl, Propyl, Fluor und/oder Chlor substituiertes Phenyl steht und außerdem

$R^4$ und $R^5$ auch gemeinsam mit dem Kohlenstoffatom, an das sie gebunden sind, für Cyclopentyl, Cyclohexyl, Cyclopentenyl oder Cyclohexenyl stehen, und

X für Stickstoff oder eine CH-Gruppe steht.

Die Racemate von Azolderivaten der Formel (Ia) sind bereits bekannt (vgl. DE-A-2 737 489, EP-A-0 015 387, EP-A-0 032 200, EP-A-0 044 425 und EP-A-0 053 311).

Die bei der Durchführung des erfindungsgemäßen Verfahrens weiterhin als Ausgangsstoffe benötigten Permethrinsäurehalogenide sind durch die Formel (II) definiert. Besonders bevorzugt sind Permethrinsäurechloride. Beispielhaft genannt seien 1R-trans-Permethrinsäurechlorid, 1S-trans-Permethrinsäurechlorid, 1R-cis-Pemethrinsäurechlorid und 1S-cis-Permetrhinsäurechlorid.

Die optisch aktiven Permethrinsäurehalogenide der Formel (II) sind bereits bekannt (vgl. EP-A-0 022 972).

Die erste Stufe des erfindungsgemäßen Verfahrens wird vorzugsweise in Gegenwart von Basen durchgeführt.

Hierbei können alle üblichen organischen oder anorganischen Basen eingesetzt werden. Vorzugsweise verwendbar sind Alkalicarbonate, wie beispielsweise Natriumcarbonat oder Natriumhydrogencarbonat, ferner niedere tertiäre Alkylamine, Cycloalkylamine, Arylalkylamine oder Arylamine, wie beispielweise Triethylamin, N,N-Dimethylbenzylamin, Pyridin, 1,4-Diazabicyclo-[2,2,2]-octan oder 1,5-Diazabicyclo-[4,3,0]-non-5-en.

Als Verdünnungsmittel kommen für die Durchführung der ersten Stufe (Veresterung) des erfindungsgemäßen Verfahrens alle inerten organischen Solventien in Frage.

Vorzugsweise verwendbar sind Kohlenwasserstoffe, wie Benzin, Benzol, Toluol oder Xylol, halogenierte Kohlenwasserstoffe, wie Dichlormethan, Chloroform, Tetrachlorkohlenstoff, Chlorbenzol oder Dichlorbenzol, Ether wie Diethylether, Tetrahydrofuran oder Dioxan, Nitrile, wie Acetonitril oder Propionitril oder Ester, wie Essigsäureethylester.

Die Reaktionstemperaturen können bei der Durchführung der 1. Stufe des erfindungsgemäßen Verfahrens in einem größeren Bereich variiert werden. Im allgemeinen arbeitet man zwischen -10°C und +150°C, vorzugsweise zwischen 0°C und 100°C.

Das erfindungsgemäße Verfahren wird in der ersten Stufe im allgemeinen unter Normaldruck durchgeführt. Es ist jedoch auch möglich, bei erhöhtem oder vermindertem Druck zu arbeiten.

Bei der Durchführung der 1. Stufe des erfindungsgemäßen Verfahrens setzt man auf 1 Mol an racemischen Azolderivat der Formel (Ia) im allgemeinen 1 bis 2 Mol, vorzugsweise 1 bis 1,5 Mol, an optisch aktivem Permethrinsäurehalogenid der Formel (II) sowie gegebenenfalls 1,5 bis 2,5, vorzugsweise 1,5 bis 2 Mol, an Säurebindemittel ein. Die Isolierung des Diastereomeren-Gemisches erfolgt nach üblichen Methoden. Im allgemeinen geht man so vor, daß man nach beendeter Umsetzung mit Wasser versetzt, die organische Phase abtrennt und nach dem Trocknen unter vermindertem Druck einengt.

Die Trennung der diastereomeren Ester der Formel (III) kann in der 2. Stufe des erfindungsgemäßen Verfahrens nach den für derartige Zwecke geeigneten Methoden vorgenommen werden, also zum Beispiel durch fraktionierte Kristallisation oder auch mit Hilfe von chromatographischen Verfahren. Vorzugsweise geht man in der Weise vor, daß man zunächst durch fraktionierte Kristallisation das gewünschte Produkt anreichert und anschließend auf chromatographischem Wege, wie zum Beispiel durch Hochdruckflüssigkeitschromatographie über geeignete Säulen, reinigt.

Von den beiden Diastereomeren der Formel (III) läßt sich im allgemeinen eines der beiden Produkte besonders gut durch fraktionierte Kristallisation isolieren. Handelt es sich hierbei um dasjenige Diastereomere, aus dem sich in der 3. Stufe des erfindungsgemäßen Verfahrens das gewünschte optisch aktive Azolderivat der Formel (I) in Freiheit setzen läßt, so ist die gezielte Herstellung der betreffenden Substanz erreicht. Handelt es sich jedoch um dasjenige Diastereomere, das nicht zu dem gewünschten Produkt führt, so ist die gezielte Herstellung der betreffenden Substanz zunächst nur bedingt möglich. In diesem Fall geht man zwechmäßigerweise so vor, daß man in der ersten Stufe das komplementäre optisch aktive Permethrinsäurehalogenid der Formel (II) verwendet. Dadurch wird erreicht, daß dann dasjenige Diastereomere der Formel (III) unter identischen Reaktionsbedingungen bevorzugt kristallisiert, aus dem die gewünschte Substanz in der 3. Stufe des Verfahrens in Freiheit gesetzt werden kann.

Als Verdünnungsmittel für die 2. Stufe (Esterverseifung) des erfindungsgemäßen Verfahrens kommen ebenfalls inerte organische Lösungsmittel in Frage. Besonders bevorzugt verwendet man Alkohol, wie beispielsweise Methanol, Ethanol oder Propanol.

Die Freisetzung der erfindungsgemäßen Wirkstoffe erfolgt in der zweiten Stufe des erfindungsgemäßen Verfahrens mit Hilfe von Basen. Bevorzugt verwendet man dabei wäßrige anorganische Basen, wie Natriumhydroxid oder Kaliumhydroxid in Wasser.

Die Reaktionstemperaturen können bei der Durchführung der 2. Stufe des erfindungsgemäßen Verfahrens ebenfalls in einem größeren Bereich variiert werden. Im allgemeinen arbeitet man zwischen 0°C und 120°C, vorzugsweise zwischen 10°C und 100°C.

Bei der Durchführung der 3. Stufe des erfindungsgemäßen Verfahrens setzt man auf 1 Mol des jeweiligen diastereomeren Esters der Formel (III) im allgemeinen 1 bis 3 Mol, vorzugsweise 1 bis 2 Mol Base ein.

Die Isolierung der erfindungsgemäßen Wirkstoffe erfolgt nach üblichen Methoden. Im allgemeinen verfährt man in der Weise, daß man das Reaktionsgemisch mit Wasser versetzt, dann mehrfach mit einem mit Wasser wenig mischbaren organischen Lösungsmittel extrahiert, die vereinigten organischen Phasen trocknet und durch Abziehen des Lösungsmittels unter vermindertem Druck einengt und den verbleibenden Rückstand gegebenenfalls durch Umkristallisation oder durch Waschen mit einem organischen Lösungsmittel von eventuell vorhandenen Verunreinigungen befreit.

Bei der Durchführung der dritten Stufe des erfindungsgemäßen Verfahrens wird jeweils derjenige diastereomere Ester der Formel (III) eingesetzt, aus dem durch Behandlung mit Base die gewünschte erfindungsgemäße, optisch aktive Substanz der Formel (I) in Freiheit gesetzt wird.

Die nach dem erfindungsgemäßen Verfahren herstellbaren optisch aktiven Azolderivate zeichnen sich durch sehr gute fungizide bzw. pflanzenwuchsregulierende Eigenschaften aus.

Das erfindungsgemäße Verfahren wird durch die folgenden Beispiele illustriert.

**Beispiel 1**

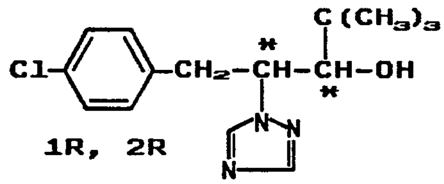

1R, 2R

(I-1)

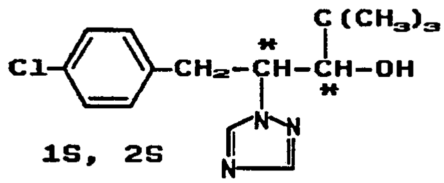

1S, 2S

(I-2)

a) **Veresterung**

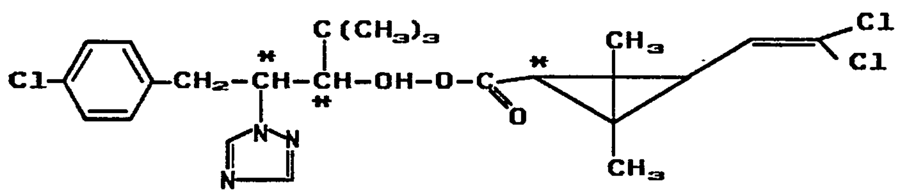

(III-1)

(Diastereomeren-Gemisch)

Ein Gemisch aus 1 g (4,4 mMol) 1S-trans-Permethrinsäurechlorid, 1,25 g (4,2 mMol) racemischem 1-(4-Chlor-phenyl)-2-(1,2,4-triazol-1-yl)-3-hydroxy-4,4-dimethyl-pentan und 10 ml Pyridin wird 2 Stunden bei 110 bis 130°C gerührt. Danach wird mit Toluol und Wasser versetzt. Die organische Phase wird abgetrennt, getrocknet und unter vermindertem Druck eingedampft. Man erhält auf diese Weise 2,1 g eines honiggelben, kristallinen Produktes, das gemäß gaschromatographischer Analyse zu 80 % aus einem Diastereomeren-Gemisch von Estern der Formel (III - 1) besteht. Demnach errechnet sich die Ausbeute zu 84 % der Theorie.

$H^1$-NMR-Spektrum (90 MHz):

δ = 0,74 und 0,80 (9H, 2s); δ = 1,24; 1,30 und 1,39 (6H, 4s);
δ = 1,75 und 1,77 (1H, 2d); δ = 2,36 (1H, 2dd);
δ = 3,0 (2H, 2d); δ = 4,72 - 4,95 (1H, m); δ = 5,09 (1H, 2d);
δ = 5,71 und 5,74 (1H, 2d); δ = 6,8 bis 7,2 (4H, d);
δ = 7,8 (1H, s); δ = 8,13 und 8,19 (1H, 2s)

b) **Trennung**

Ein Diastereomeren-Gemisch von trans-1S-Permethrinsäure-[1-(4-chlorphenyl)-2-(1,2,4-triazol-1-yl)-4,4-dimethyl-pent-3-yl]-estern (vgl. a) wird getrennt, indem man eine 20 Gew.-%-ige Lösung des Diastereomeren-Gemisches in Chloroform chromatographiert.

| | |
|---|---|
| Geräte: | Lobarsystem bestehend aus: |
| Pumpe: | CFG Typ 1pr |
| Detektor: | Knauer RJ-Detektor |
| Autofraktionssammler: | LKB 2111 Multirac |
| Eluierungsmittel: | n-Hexan: tert.-Butyl-methyl-Ether = 6 : 4 |
| Fließgeschwindigkeit: | 6 ml pro Minute |
| Säule: | Lichropräp. Si 60, Größe C |
| Aufgabemenge: | 4 g |
| Retentionszeit: | $t_R$ = 4 - 5,5 h |

Die aufgefangenen Fraktionen mit identischem Inhalt werden vereinigt und eingedampft. Man erhält auf diese Weise jedes der beiden Diastereomeren in einer Ausbeute von 70 % der Theorie. Die chemische Reinheit beträgt jeweils über 98 %; die optische Reinheit liegt bei 98 %.

Gaschromatographie:

50 m SE 30 Glaskapillare
2 ml Helium min
120°C/12°C $min^{-1}$ / 300° C; 21' isotherm

1S,2S-1S-trans-Ester (Fraktion 1):

| | |
|---|---|
| $t'_R$ = 22,4' | 98 % |
| $t''_R$ = 22,7' | 2 % |

1R,2R-1S-trans-Ester (Fraktion 2):

| | |
|---|---|
| $t'_R$ = 22,4' | 2 % |
| $t''_R$ = 22,7' | 98 % |

**c) Verseifung**

Es werden jeweils 1 g eines der zuvor genannten Ester (vgl. b) und 30 ml 5 gew.-%-iger methanolischer Kalilauge 5 Stunden unter Rückfluß erhitzt. Danach wird das Reaktionsgemisch unter vermindertem Druck bei 40°C eingedampft. Der verbleibende Rückstand wird mit Wasser und Diethylether versetzt. Man trennt die organische Phase ab, wäscht mit Wasser, trocknet und engt unter vermindertem Druck ein. Man erhält auf diese Weise 0,6 g (91 % der Theorie) an der jeweiligen Verbindung in Form eines kristallinen Produktes. Die chemische Reinheit beträgt - 98 %; die optische Reinheit liegt bei - 96 % ee (nach gaschromatischer Auswertung bezogen auf 100 % - Fläche der Diastereomeren-Permethrinsäureester). 1S,2S-Verbindung:

$[\alpha]^{=3}$ = +80° (C = 0,015 Mol/l; $CHCl_3$)

1R,2R-Verbindung:

$[\alpha]^{23}_D$ = -80° (C = 0,015 Mol/l; $CHCl_3$)

**Beispiel 2**

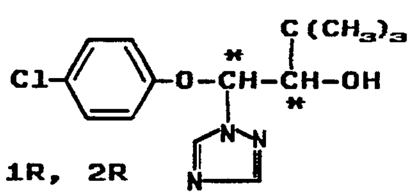

(I-3)

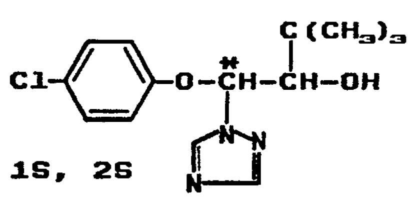

(I-4)

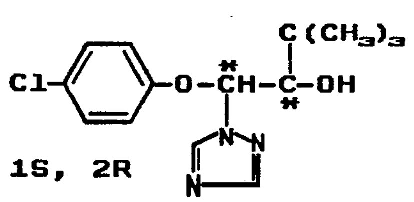

(I-5)

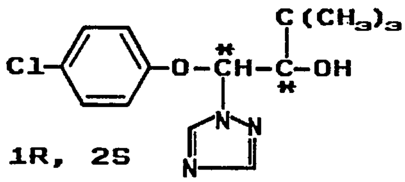

(I-6)

**a) Veresterung**

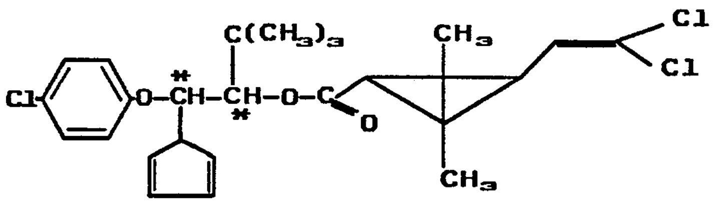

(III-2)

Diastereomeren-Gemisch

Ein Gemisch aus 1 g (4,4 mMol) 1S-trans-Permethrinsäurechlorid, 1,25 g (4,2 mMol) 1-(4-Chlorphenoxy)-1-(1,2,4-triazol-1-yl)-2-hydroxy-3,3-dimethyl-butan (Gemisch aus 50 % threo 1R, 2S und 1S, 2R sowie 50 % erythro 1R, 2R und 1S, 2S) wird 3 Stunden bei 100°C bis 110°C gerührt. Danach werden zunächst 5 ml Triethylamin hinzugefügt, und dann wird mit Toluol und Wasser versetzt. Die organische Phase wird abgetrennt, mit Wasser gewaschen, getrocknet und unter vermindertem Druck eingedampft. Man erhält auf diese Weise 2,1 g eines kristallinen Produktes, das gemäß gaschromatographischer Analyse zu 90 % aus einem Diastereomeren-Gemisch von Estern der Formel (III - 2) besteht. Danach errechnet sich die Ausbeute zu 93 % der Theorie.

**b) Trennung**

Ein Diastereomeren-Gemisch von trans-1S-Permethrinsäure-[1-(4-chlorphenoxy)-1-(1,2,4-triazol-1-yl)-3,3-dimethyl-but-2-yl]-estern (vgl. a) wird getrennt, indem man 1,1 ml einer 10 %-igen Lösung des Diastereomeren-Gemisches in Cyclohexan/Methylenchlorid = 85 : 15 chromatographiert.

| | | |
|---|---|---|
| Geräte: | Perkin-Elmer Autosampler, Mo · 420<br>Perkin-Elmer LC-Chromatograph, Serie 3B<br>Perkin-Elmer UV-Detektor LC 75<br>Autofraktionssammler Gilson CPR Ref 45.500 | |
| Eluierungsmittel: | Cyclohexan-Methylenchlorid 85:15 | |
| Fließgeschwindigkeit: | 15 ml $min^{-1}$ (2 Pumpen) | |
| Säule: | Lichrosorb Diol 5 µ ø 2,5 cm | |
| Retentionszeiten: | $t_R$ + threo-Ester | 14 min |
| | $t_R$ - threo-Ester | 13 " |
| | $t_R$ + erythro-Ester | 18 " |
| | $t_R$ - erythro-Ester | 16 " |

Sämtliche Fraktionen werden über die Peakhöhenansteuerung am Gilson-Sammler zwischen 60 und 80 % "Höhe" aufgefangen. Das Lösungsmittel wird jeweils unter vermindertem Druck abgezogen. Es verbleibt jeweils ein weißer, kristalliner Rückstand. Die Struktur der isolierten Substanzen wird durch NMR-spektroskopische Daten gesichert. Die optische Reinheit jeder Fraktion, die durch Kapillar-Gaschromatographie überprüft wurde, ist jeweils größer als 98 %.

**Gaschromatographie:**

| | |
|---|---|
| 50 m | S 30 Glaskapillare |
| 2 ml | Helium/min |
| 200° C/8° C $min^{-1}$/300°C | |
| $t'_R$ | = 9,40 min |
| $t''_R$ | = 9,55 min |
| $t'''_R$ | = 9,64 min |
| $t''''_R$ | = 9,70 min |

**Isomeres A (threo):**

$H^1$-NMR-Spektrum (90 MHz)

δ = 1,05 (9H, s); δ = 1,14 (3H, s);
δ = 1,21 (3H, s); δ = 1,70 (1H, d); δ = 2,17 (1H, d);
δ = 5,17 (1H, d); δ = 5,64 (1H, d); δ = 6,38 (1H,d);
δ = 6,78 (2H, arom.); δ = 7,17 (2H, arom.);
δ = 7,94 (1H, s); δ = 8,19 (1H, s).

**Isomeres B (threo):**

$H^1$-NMR-Spektrum (90 MHz)

δ = 0,98 (9H, s); δ = 1,21 (3H, s); δ = 1,27 (3H, s);
δ = 1,66 (1H, d); δ = 2,23 (1H, dd);
δ = 5,21 (1H, d); δ = 5,61 (1H, d);
δ = 6,30 (1H, d); δ = 6,70 - 7,25 (4H, arom.)
δ = 7,95 (1H, s); δ = 8,21 (1H, s).

**Isomeres C (erythro):**

$H^1$-NMR-Spektrum (90 MHz)

δ = 0,95 (9H, s); δ = 1,19 (3H, s); δ = 1,24 (3H, s);
δ = 1,53 (1H, d); δ = 2,17 (1H, dd); δ = 5,39 (1H, d);
δ = 5,62 (1H, d); δ = 6,26 (1H, d);
δ = 6,7 - 7,2 (4H, arom.);
δ = 7,90 (1H, s); δ = 8,34 (1H,s).

**Isomeres D (erythro):**

$H^1$-NMR-Spektrum (90 MHz)

δ = 0,98 (9H, s); δ = 1,17 (6H, s); δ = 1,54 (1H, d);
δ = 2,19 (1H, dd); δ = 5,40 (1H, d); δ = 5,62 (1H, d);
δ = 6,26 (1H, d); δ = 6,70 - 7,28 (4 H, arom.)
δ = 7,93 (1H, s); δ = 8,32 (1H, s).

**c) Verseifung**

Ein Gemisch aus dem jeweiligen trans-1S-Permethrinsäure-[1-(4-chlorphenoxy)-1-(1,2,4-triazol-1-yl)-3,3-dimethyl-but-2-yl]-ester (vgl. b) und 100 ml 5 gew.-%-iger methanolischer Kalilauge wird 1,5 Stunden unter Rückfluß erhitzt. Danach wird das Reaktionsgemisch unter vermindertem Druck bei 40°C eingedampft. Der verbleibende Rückstand wird mit Wasser und Diethylether versetzt. Man trennt die organische Phase ab, wäscht mit Wasser, trocknet und engt unter vermindertem Druck ein. Auf diese Weise erhält man das jeweils gewünschte Produkt in Form eines kristallinen Stoffes.

**Beispiel 3**

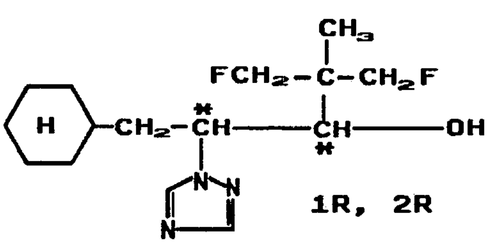

1R, 2R

(I-7)

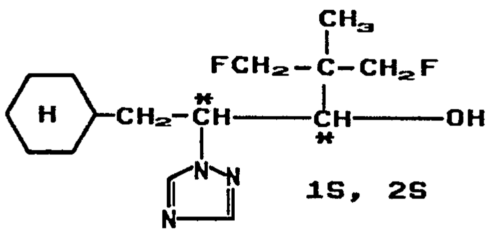

(I-8)

a) Veresterung

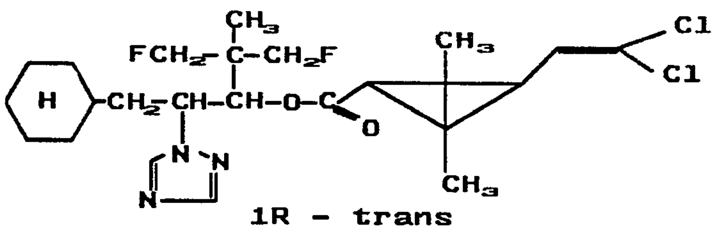

(III-3)

Diastereomeren-Gemisch

Ein Gemisch aus 760 mg (3,3 mMol) 1R-trans-Permethrinsäurechlorid und 1 g (3,3 mMol) racemischem 1-Cyclohexyl-2-(1,2,4-triazol-1-yl)-3-hydroxy-4,4-bis-fluormethyl-pentan wird 12 Stunden bei 100°C gerührt. Danach wird mit 5 ml Triethylamin versetzt. Zu dem resultierenden Reaktionsgemisch werden Diethylether und Wasser hinzugefügt. Die organische Phase wird abgetrennt, getrocknet und unter vermindertem Druck eingedampft. Man erhält auf diese Weise 1,5 g eines honiggelben Produktes, das gemäß Gaschromatogramm zu 92 % aus einem Diastereomerengemisch von Verbindungen der oben angegebenen Formel (III - 3) besteht. Danach errechnet sich die Ausbeute zu 87 % der Theorie.

$H^1$ - Kernresonanzspektrum (90 MHz)]

δ = 0,57 (3H, sbr.); δ = 0,8 - 1,8 (20H, mbr.);
δ = 2,2 - 2,3 (1H, 2dd); δ = 3,8 - 4,8 (5H, mbr.);
δ = 5,2 (1H, d); δ = 5,7 und 5,8 (1H, 2d);
δ = 7,9 (1H, s);
δ = 8,14 und 8,20 (1H, 2s).

b) Trennung

Ein Diastereomeren-Gemisch von 1R-trans-Permethrinsäure-[1-cyclohexyl-2-(1,2,4-triazol-1-yl)-4,4-bis-fluormethyl-pent-3-yl]-estern (vgl. a) wird getrennt, indem man eine 30 %-ige Lösung des Diastereomeren-Gemisches in n-Hexan/Diethylether = 6 : 4 chromatographiert.

| | |
|---|---|
| Geräte: | Lobarsystem bestehend aus: |
| Pumpe: | CFG typ : lpr |
| Detektor: | Knauer RI-Detektor |
| | Autofraktionssammler: |
| | LKB 2111 Multirac |
| Eluierungsmittel: | n-Hexan: Diethylether = 6 : 4 |
| Fließgeschwindigkeit: | 6 ml $min^{-1}$ |
| Säule: | Lichropräp. Si 60 Größe C |
| Aufgabemenge: | 1,5 - 2,0 g |
| Retentionszeit: | $t_R$ = 3 - 3,5 Stunden |

Das eluierte Produkt mit einem Gehalt von 99 % an dem gewünschten Diastereomeren wird aus den Fraktionen nach der Peakfronting-Methode gewonnen. Die Fraktionen mit einem Gehalt von 60 bis 99 % an "Ziel-Diastereomerem" werden einer nochmaligen Trennung unterworfen. Man erhält nach dieser Arbeitsweise 40 % des eingesetzten Diastereomeren-Esters mit einer chemischen Reinheit von > 98 % und einer optischen Reinheit von > 99 %. Dabei wird die optische Reinheit durch Kapillar-Gaschromatographie ermittelt.

Gaschromatographie:

| | |
|---|---|
| 50 ml | SE 30 Glaskapillare |
| 2 ml | Helium/min |

120°C/12°C $min^{-1}$/300°C/21' isotherm

t'$_R$ = 21,5' > 99 %
t"$_R$ = 21,9' < 1 %

**c) Verseifung**

Ein Gemisch aus 1 g an dem jeweiligen 1R-trans-Permethrinsäure-[1-cyclohexyl-2-(1,2,4-triazol-1-yl)-4,4-bis-fluormethyl-pent-3-yl]-ester (vgl. b) in 10 ml 5 gew.-%-iger methanolischer Kalilauge wird 1 Stunde bei 40°C gerührt. Danach wird das Reaktionsgemisch unter vermindertem Druck eingeengt. Der verbleibende Rückstand wird mit Wasser und 20 ml Diethylether versetzt. Man trennt die organische Phase ab, wäscht mit Wasser, trocknet und engt unter vermindertem Druck ein. Auf diese Weise erhält man 0,57 g (91 % der Theorie) an der gewünschten Verbindung in Form eines kristallinen Produktes. Die chemische Reinheit beträgt > 98 %; die optische Reinheit liegt > 98 % ee (nach gaschromatographischer Auswertung bezogen auf 100 % Fläche der Diastereomeren-Permethinsäureester).

$[\alpha]^{23}_D$ = -22,2° (c = 0,038 Mol/l; $CHCl_3$)
$[\alpha]^{23}_D$ = +22,6° (c = 0,039 Mol/l; $CHCl_3$)*)

*) Die Darstellung des (+)-Isomeren erfolgte durch Einsatz von 1S-trans-Permethrinsäurechlorid und identischem Arbeiten.

Nach den oben angegebenen Methoden wurden auch die in den folgenden Beispielen formelmäßig aufgeführten optisch aktiven Azol-Derivate hergestellt.

**Beispiel 4**

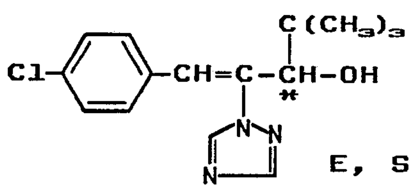

(I-9)

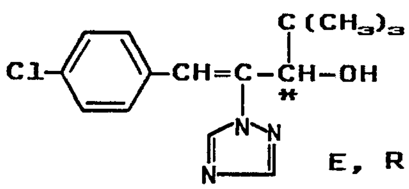

(I-10)

**Beispiel 5**

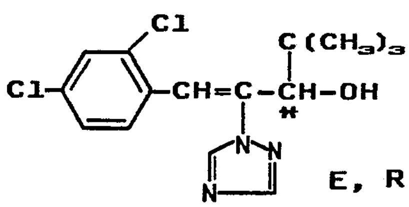

(I-11)

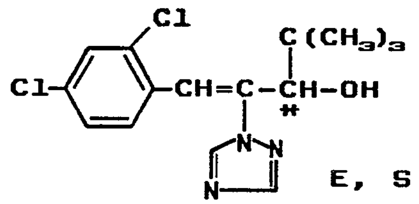

(I-12)

## Patentansprüche

1. Verfahren zur Herstellung von optisch aktiven Azolderivaten der Formel

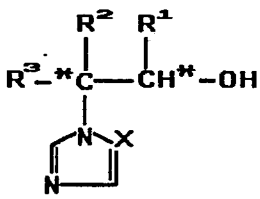

(I)

in welcher

$R^1$ für geradkettiges oder verzweigtes Alkyl mit 1 bis 4 Kohlenstoffatomen, für gegebenenfalls einfach oder mehrfach durch Alkyl mit 1 bis 2 Kohlenstoffatomen substituiertes Cycloalkyl mit 3 bis 7 Ringkohlenstoffatomen sowie für Phenyl oder Naphthyl steht, wobei jeder der beiden zuvor genannten Reste einfach oder mehrfach, gleichartig oder verschieden substituiert sein kann durch Halogen, geradkettiges oder verzweigtes Alkyl mit 1 bis 4 Kohlenstoffatomen sowie Halogenalkyl mit 1 oder 2 Kohlenstoffatomen und 1 bis 5 gleichen oder verschiedenen Halogenatomen, wie Fluor- und Chloratomen, und

$R^1$ weiterhin für einen 5- oder 6-gliedrigen, gegebenenfalls benzannellierten Heterocyclus mit 1 bis 3 gleichen oder verschiedenen Heteroatomen, wie Sauerstoff, Stickstoff und Schwefel steht, wobei der Heterocyclus einfach oder mehrfach, gleichartig oder verschieden substituiert sein kann durch Halogen, Alkyl mit 1 bis 4 Kohlenstoffatomen oder Halogenalkyl mit 1 oder 2 Kohlenstoffatomen und 1 bis 5 gleichen oder verschiedenen Halogenatomen, wie Fluor- oder Chloratomen, und

$R^1$ außerdem für die Reste der Formeln

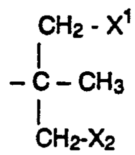

oder

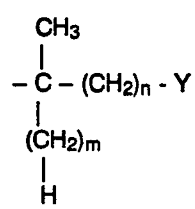

steht, wobei

$X^1$ für Wasserstoff oder Halogen steht,

$X^2$ für Halogen steht,

Y für geradkettiges oder verzweigtes Alkyl mit 1 bis 6 Kohlenstoffatomen, Alkoxy mit 1 bis 6 Kohlenstoffatomen, Alkylthio mit 1 bis 6 Kohlenstoffatomen, Halogenalkoxy mit 1 bis 6 Kohlenstoffatomen und 1 bis 9 gleichen oder verschiedenen Halogenatomen, Halogenalkylthio mit 1 bis 6 Kohlenstoffatomen und 1 bis 9 gleichen oder verschiedenen Halogenatomen, Alkenyl mit 2 bis 6 Kohlenstoffatomen, geradkettiges oder verzweigtes Alkoxycarbonyl mit 1 bis 4 Kohlenstoffatomen im Alkoxyteil, Cyano sowie für Phenyl, Phenyloxy, Phenylthio, Phenylalkoxy mit 1 bis 4 Kohlenstoffatomen in der Alkoxygruppe und Phenylalkylthio mit 1 bis 4 Kohlenstoffatomen in der Alkylthiogruppe steht, wobei jeder der zuvor genannten Phenyl-Reste einfach oder mehrfach, gleichartig oder verschieden substituiert sein kann durch Halogen, Alkyl mit 1 bis 4 Kohlenstoffatomen, Alkoxy mit 1 bis 2 Kohlenstoffatomen, Alkylthio mit 1 oder 2 Kohlenstoffatomen, Halogenalkyl mit 1 oder 2 Kohlenstoffatomen und 1 bis 5 gleichen oder verschiedenen Halogenatomen, wie Fluor- und Chloratomen, Halogenalkoxy mit 1 oder 2 Kohlenstoffatomen und 1 bis 5 gleichen oder verschiedenen Halogenatomen, wie Fluor- und Chloratomen, Halogenalkylthio mit 1 oder 2 Kohlenstoffatomen und 1 bis 5 gleichen oder verschiedenen Halogenatomen, wie Fluor- und Chloratomen, Cycloalkyl mit 3 bis 7 Kohlenstoffatomen, Dialkylamino mit 1 bis 4 Kohlenstoffatomen in jedem Alkylteil, Cyano, Nitro sowie geradkettiges oder verzweigtes Alkoxycarbonyl mit 1 bis 4 Kohlenstoffatomen im Alkoxyteil, und

m für eine Zahl 0 oder 1 steht,

n für eine Zahl 0, 1 oder 2 steht,

$R^2$ für Wasserstoff oder Methyl steht,

$R^3$ für Alkyl mit 1 bis 6 Kohlenstoffatomen, Alkoxy mit 1 bis 6 Kohlendstoffatomen, Cycloalkylalkyl mit 3 bis 8 Kohlenstoffatomen in der Cycloalkylgruppe und 1 bis 4 Kohlenstoffatomen im Alkylteil, gegebenenfalls ein- oder mehrfach, gleichartig oder verschieden durch Alkyl mit 1 bis 4 Kohlenstoffatomen, Halogen, Phenyl und/oder Nitro substituiertes Aryl mit 6 bis 10 Kohlenstoffatomen, gegebenenfalls ein- oder mehrfach, gleichartig oder verschieden durch Alkyl mit 1 bis 4 Kohlenstoffatomen, Halogen, Phenyl und/oder Nitro substituiertes Aralkyl mit 6 bis 10 Kohlenstoffatomen im Arylteil und 1 bis 4 Kohlenstoffatomen im Alkylteil, für gegebenenfalls ein- oder mehrfach, gleichartig oder verschieden durch Alkyl mit 1 bis 4 Kohlenstoffatomen, Halogen, Phenyl und/oder Nitro substituiertes Aroxy mit 6 bis 10 Kohlenstoffatomen oder für gegebenenfalls ein- oder mehrfach, gleichartig oder verschieden durch Alkyl mit 1 bis 4 Kohlenstoffatomen, Halogen, Phenyl und/oder Nitro substituiertes Aroxyalkyl mit 6 bis 10 Kohlenstoffatomen im Aroxyteil und 1 bis 4 Kohlenstoffatomen im Alkylteil steht, oder

$R^2$ und $R^3$ auch gemeinsam für die Gruppe

```
  R4
 /
=C    stehen, in welcher
 \
  R5
```

$R^4$ für Wasserstoff, Alkyl mit 1 bis 4 Kohlenstoffatomen, Cycloalkyl mit 3 bis 8 Kohlenstoffatomen oder Cycloalkenyl mit 5 bis 8 Kohlenstoffatomen steht und

$R^5$ für Alkyl mit 1 bis 4 Kohlenstoffatomen, Cycloalkyl mit 3 bis 8 Kohlenstoffatomen, Cycloalkenyl mit 5 bis 8 Kohlenstoffatomen oder für gegebenenfalls ein- oder mehrfach, gleichartig oder verschieden durch Alkyl mit 1 bis 4 Kohlenstoffatomen und/oder Halogen substituiertes Aryl mit 6 bis 10 Kohlenstoffatomen steht oder

$R^4$ und $R^5$ gemeinsam mit dem Kohlenstoffatom, an das sie gebunden sind, für Cycloalkyl mit 5 bis 8 Kohlenstoffatomen oder für Cycloalkenyl mit 5 bis 8 Kohlenstoffatomen stehen und

X für Stickstoff oder eine CH-Gruppe steht, dadurch gekennzeichnet, daß man in einer ersten Stufe Racemate von Azolderivaten der Formel

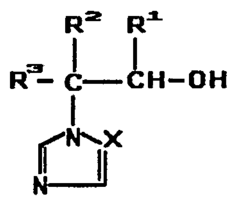

(Ia)

in welcher

$R^1$, $R^2$, $R^3$ und X die oben angegebene Bedeutung haben,

mit optischen aktiven Permethrinsäurehalogeniden der Formel

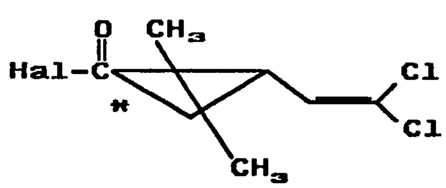

(II)

in welcher

Hal für Chlor oder Brom steht,

gegebenenfalls in Gegenwart eines Säurebindemittels und gegebenenfalls in Gegenwart eines Verdünnungsmittels umsetzt, dann in einer zweiten Stufe die so erhaltenen diastereomeren Ester der Formel

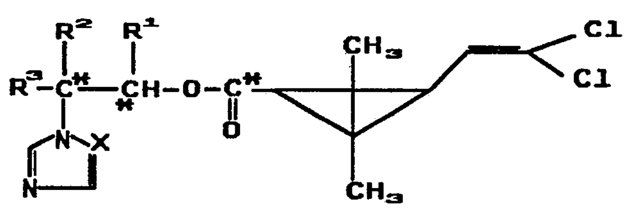

(III)

(Diastereomeren-Gemisch)

in welcher
$R^1$, $R^2$, $R^3$ und X die oben angegebene Bedeutung haben,
aufgrund ihrer unterschiedlichen physikalischen Eigenschaften trennt
und danach in einer dritten Stufe das jeweilige Azolderivat der Formel (I) aus dem entsprechenden Ester mit Hilfe von Basen in Gegenwart eines Verdünnungsmittels in Freiheit setzt.

2. Verfahren gemäß Anspruch 1, dadurch gekennzeichnet, daß man als optisch aktives Permethrinsäurehalogenid der Formel (II) 1R-trans-Permethrinsäurechlorid einsetzt.

3. Verfahren gemäß Anspruch 1, dadurch gekennzeichnet, daß man als optisch aktives Permethrinsäurehalogenid der Formel (II) 1S-trans-Permethrinsäurechlorid einsetzt.

4. Verfahren gemäß Anspruch 1, dadurch gekennzeichnet, daß man bei der Durchführung der ersten Stufe bei Temperaturen zwischen -10°C und +150°C arbeitet.

5. Verfahren gemäß Anspruch 1, dadurch gekennzeichnet, daß man bei der Durchführung der dritten Stufe bei Temperaturen zwischen 0°C und 120°C arbeitet.

6. Verfahren gemäß Anspruch 1, dadurch gekennzeichnet, daß man bei der Durchführung der ersten Stufe auf 1 Mol an racemischem Azolderivat der Formel (Ia) 1 bis 2 Mol an optisch aktivem Permethrinsäurehalogenid der Formel (II) und 1,5 bis 2,5 Mol an Säurebindemittel einsetzt.

## Claims

1. Process for the preparation of optically active azole derivatives of the formula

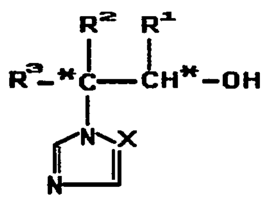

(I)

in which

$R^1$ represents straight-chain or branched alkyl with 1 to 4 carbon atoms, or represents cycloalkyl which has 3 to 7 ring carbon atoms and is optionally monosubstituted or polysubstituted by alkyl with 1 or 2 carbon atoms, or represents phenyl or naphthyl, it being possible for each of the above two radicals mentioned to be monosubstituted or polysubstituted by identical or different substituents from the group comprising halogen, straight-chain or branched alkyl with 1 to 4 carbon atoms and halogenoalkyl with 1 or 2 carbon atoms and 1 to 5 identical or different halogen atoms, such as fluorine and chlorine atoms, or

$R^1$ furthermore represents a 5-membered or 6-membered, optionally benzo-fused heterocyclic radical with 1 to 3 identical or different hetero atoms, such as oxygen, nitrogen and sulphur, it being possible for the heterocyclic radical to be monosubstituted or polysubstituted by identical or different substituents from the group comprising halogen, alkyl with 1 to 4 carbon atoms and halogenoalkyl with 1 or 2 carbon atoms and 1 to 5 identical or different halogen atoms, such as fluorine or chlorine atoms, or

$R^1$ furthermore represents the radicals of the formulae

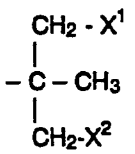

or

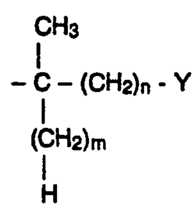

wherein

$X^1$ represents hydrogen or halogen,
$X^2$ represents halogen,
Y represents straight-chain or branched alkyl with 1 to 6 carbon atoms, alkoxy with 1 to 6 carbon atoms, alkylthio with 1 to 6 carbon atoms, halogenoalkoxy with 1 to 6 carbon atoms and 1 to 9 identical or different halogen atoms,

halogenoalkylthio with 1 to 6 carbon atoms and 1 to 9 identical or different halogen atoms, alkenyl with 2 to 6 carbon atoms, straight-chain or branched alkoxycarbonyl with 1 to 4 carbon atoms in the alkoxy part or cyano, or represents phenyl, phenoxy, phenylthio, phenylalkoxy with 1 to 4 carbon atoms in the alkoxy group or phenylalkylthio with 1 to 4 carbon atoms in the alkylthio group, it being possible for each of the abovementioned phenyl radicals to be monosubstituted or polysubstituted by identical or different substituents from the group comprising halogen, alkyl with 1 to 4 carbon atoms, alkoxy with 1 or 2 carbon atoms, alkylthio with 1 or 2 carbon atoms, halogenoalkyl with 1 or 2 carbon atoms and 1 to 5 identical or different halogen atoms, such as fluorine and chlorine atoms, halogenoalkoxy with 1 or 2 carbon atoms and 1 to 5 identical or different halogen atoms, such as fluorine and chlorine atoms, halogenoalkylthio with 1 or 2 carbon atoms and 1 to 5 identical or different halogen atoms, such as fluorine and chlorine atoms, cycloalkyl with 3 to 7 carbon atoms, dialkylamio with 1 to 4 carbon atoms in each alkyl part, cyano, nitro and straight-chain or branched alkoxycarbonyl with 1 to 4 carbon atoms in the alkoxy part, and

m represents number 0 or 1,
n represents number 0, 1 or 2,
$R^2$ represents hydrogen or methyl,
$R^3$ represents alkyl with 1 to 6 carbon atoms, alkoxy with 1 to 6 carbon atoms, cycloalkylalkyl with 3 to 8 carbon atoms in the cycloalkyl group and 1 to 4 carbon atoms in the alkyl part, aryl which has 6 to 10 carbon atoms and is optionally mono- or polysubstituted by identical or different substituents from the group comprising alkyl with 1 to 4 carbon atoms, halogen, phenyl and/or nitro, or aralkyl which has 6 to 10 carbon atoms in the aryl part and 1 to 4 carbon atoms in the alkyl part and is optionally mono- or polysubstituted by identical or different substituents from the group comprising alkyl with 1 to 4 carbon atoms, halogen, phenyl and/or nitro, or represents aroxy which has 6 to 10 carbon atoms and is optionally mono- or polysubstituted by identical or different substituents from the group comprising alkyl with 1 to 4 carbon atoms, halogen, phenyl and/or nitro, or represents aroxyalkyl which has 6 to 10 carbon atoms in the aroxy part and 1 to 4 carbon atoms in the alkyl part and is optionally mono- or polysubstituted by identical or different substituents from the group comprising alkyl with 1 to 4 carbon atoms, halogen, phenyl and/or nitro, or $R^2$ and $R^3$ also together represent the group

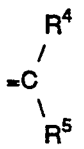

in which

$R^4$ represents hydrogen, alkyl with 1 to 4 carbon atoms, cycloalkyl with 3 to 8 carbon atoms or cycloalkenyl with 5 to 8 carbon atoms and
$R^5$ represents alkyl with 1 to 4 carbon atoms, cycloalkyl with 3 to 8 carbon atoms, cycloalkenyl with 5 to 8 carbon atoms, or aryl which has 6 to 10 carbon atoms and is optionally mono- or polysubstituted by identical or different substituents from the group comprising alkyl with 1 to 4 carbontoms and/or halogen, or
$R^4$ and $R^5$, together with the carbon atom to which they are bonded, represent cycloalkyl with 5 to 8 carbon atoms, or represent cycloalkenyl with 5 to 8 carbon atoms, and
X represents nitrogen or a CH group,

characterized in that, in a first stage, racemates of azole derivatives of the formula

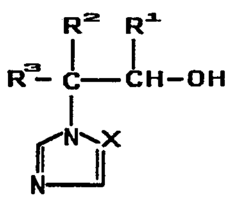

(Ia)

in which
$R^1$, $R^2$, $R^3$ and X have the abovementioned meaning, are reacted with optically active permethric acid halides of the formula

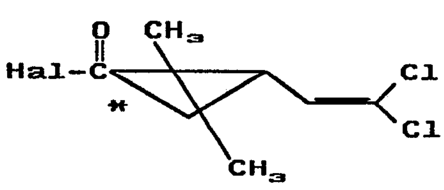

(II)

in which

Hal represents chlorine or bromine, if appropriate in the presence of an acid-binding agent and if appropriate in the presence of a diluent, and then, in a second stage, the diastereomeric esters thus obtained, of the formula

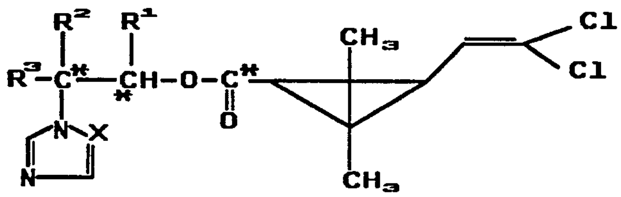

(III)

(diastereomer mixture)

in which
$R^1$, $R^2$, $R^3$ and X have the abovementioned meaning, are separated on the basis of their different physical properties, and, thereafter, in a third stage, the particular azole derivative of the formula (I) is liberated from the corresponding ester with the aid of bases in the presence of a diluent.

2. 1R-trans-permethric acid chloride is employed as the optically active permethric acid halide of the formula (II).

3. Process according to Claim 1, characterized in that 1S-trans-permethric acid chloride is used as the optically active permethric acid halide of the formula (II).

4. Process according to Claim 1, characterized in that the first stage is carried out at temperatures between -10°C and +150°C.

5. Process according to Claim 1, characterized in that the third stage is carried out at temperatures between 0°C and 120°C.

6. Process according to Claim 1, characterized in that in carrying out the first stage, 1 to 2 moles of optically active permethric acid halide of the formula (II) and 1.5 to 2.5 moles of acid-binding agent are employed per mole of racemic azole derivative of the formula (Ia).

**Revendications**

1. Procédé de production de dérivés azoliques optiquement actifs de formule

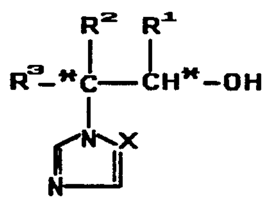

(I)

dans laquelle

$R^1$ est un groupe alkyle à chaîne droite ou à chaîne ramifiée ayant 1 à 4 atomes de carbone, un groupe cycloalkyle de 3 à 7 atomes de carbone sur le noyau, portant éventuellement un ou plusieurs substituants alkyle de un ou deux atomes de carbone, ainsi qu'un groupe phényle ou naphtyle, chacun des deux restes précédemment mentionnés pouvant porter un ou plusieurs substituants, identiques ou différents, halogéno, alkyle à chaîne droite ou ramifiée ayant 1 à 4 atomes de carbone ainsi qu'halogénalkyle ayant 1 ou 2 atomes de carbone et 1 à 5 atomes d'halogènes identiques ou différents, tels que des atomes de fluor et de chlore, et
$R^1$ désigne en outre un hétérocycle pentagonal ou hexagonal éventuellement condensé au benzène, comprenant 1 à 3 hétéroatomes identiques ou différents tels qu'oxygène, azote et soufre, l'hétérocycle pouvant porter un ou plusieurs substituants, identiques ou différents, halogéno, alkyle ayant 1 à 4 atomes de carbone ou halogénalkyle ayant 1 ou 2 atomes de carbone et 1 à 5 atomes d'halogènes identiques ou différents tels que des atomes de fluor ou de chlore, et
$R^1$ représente en outre les restes de formules

$$\begin{array}{l} CH_2-X^1 \\ | \\ -C-CH_3 \\ | \\ CH_2-X^2 \end{array} \quad \text{ou} \quad \begin{array}{l} CH_3 \\ | \\ -C-(CH_2)_n-Y \\ | \\ (CH_2)_m \\ | \\ H \end{array}$$

dans lesquelles

$X^1$ est l'hydrogène ou un halogène,

$X^2$ est un halogène,

Y est un groupe alkyle à chaîne droite ou à chaîne ramifiée ayant 1 à 6 atomes de carbone, un groupe alkoxy ayant 1 à 6 atomes de carbone, un groupe alkylthio ayant 1 à 6 atomes de carbone, un groupe halogénalkoxy ayant 1 à 6 atomes de carbone et 1 à 9 atomes d'halogènes identiques ou différents, un groupe halogénalkylthio ayant 1 à 6 atomes de carbone et 1 à 9 atomes d'halogènes identiques ou différents, un groupe alcényle de 2 à 6 atomes de carbone, un groupe alkoxycarbonyle à chaîne droite ou ramifiée ayant 1 à 4 atomes de carbone dans la partie alkoxy, un groupe cyano, ainsi qu'un groupe phényle, phényloxy, phénylthio, phénylalkoxy ayant 1 à 4 atomes de carbone dans le groupe alkoxy et un groupe phénylalkylthio ayant 1 à 4 atomes de carbone dans le groupe alkylthio, chacun des restes phényle précédemment mentionnés pouvant porter un ou plusieurs substituants, identiques ou différents, halogéno, alkyle ayant 1 à 4 atomes de carbone, alkoxy ayant 1 ou 2 atomes de carbone, alkylthio ayant 1 ou 2 atomes de carbone, halogénalkyle ayant 1 ou 2 atomes de carbone et 1 à 5 atomes d'halogènes identiques ou différents tels que des atomes de fluor et de chlore, halogénalkoxy ayant 1 ou 2 atomes de carbone et 1 à 5 atomes d'halogènes identiques ou différents tels que des atomes de fluor et de chlore, halogénalkylthio ayant 1 ou 2 atomes de carbone et 1 à 5 atomes d'halogènes identiques ou différents tels que des atomes de fluor et de chlore, cycloalkyle ayant 3 à 7 atomes de carbone, dialkylamino ayant 1 à 4 atomes de carbone dans chaque partie alkyle, cyano, nitro, ainsi qu'un groupe alkoxycarbonyle à chaîne droite ou à chaîne ramifiée ayant 1 à 4 atomes de carbone dans la partie alkoxy, et

m est le nombre 0 ou 1,

n est le nombre 0, 1 ou 2,

$R^2$ est l'hydrogène ou un groupe méthyle,

$R^3$ est un groupe alkyle de 1 à 6 atomes de carbone, un groupe alkoxy de 1 à 6 atomes de carbone, un groupe cycloalkylalkyle de 3 à 8 atomes de carbone dans le groupe cycloalkyle et de 1 à 4 atomes de carbone dans la partie alkyle, un groupe aryle de 6 à 10 atomes de carbone portant éventuellement un ou plusieurs substituants, identiques ou différents, alkyle de 1 à 4 atomes de carbone, halogéno, phényle et/ou nitro, un groupe aralkyle de 6 à 10 atomes de carbone dans la partie aryle et de 1 à 4 atomes de carbone dans la partie alkyle, portant le cas échéant un ou plusieurs substituants, identiques ou différents, alkyle de 1 à 4 atomes de carbone, halogéno, phényle et/ou nitro, un groupe aroxy de 6 à 10 atomes de carbone portant éventuellement un ou plusieurs substituants, identiques ou différents, alkyle de 1 à 4 atomes de carbone, halogéno, phényle et/ou nitro, un groupe aroxyalkyle de 6 à 10 atomes de carbone dans la partie aroxy et de 1 à 4 atomes de carbone dans la partie alkyle portant éventuellement un ou plusieurs substituants, identiques ou différents, alkyle de 1 à 4 atomes de carbone, halogéno, phényle et/ou nitro, ou bien

$R^2$ et $R^3$ forment aussi conjointement le groupe

$$\begin{array}{l} \quad R^4 \\ \quad / \\ =C \\ \quad \backslash \\ \quad R^5 \end{array}$$ dans lequel

$R^4$ est l'hydrogène, un radical alkyle de 1 à 4 atomes de carbone, cycloalkyle de 3 à 8 atomes de carbone ou cycloalcényle de 5 à 8 atomes de carbone et

$R^5$ est un groupe alkyle de 1 à 4 atomes de carbone, cycloalkyle de 3 à 8 atomes de carbone, cycloalcényle de 5 à 8 atomes de carbone ou un groupe aryle de 6 à 10 atomes de carbone portant éventuellement un ou plusieurs substituants, identiques ou différents, alkyle de 1 à 4 atomes de carbone et/ou halogéno,

$R^4$ et $R^5$ forment conjointement avec l'atome de carbone auquel ils sont liés un groupe cycloalkyle de 5 à 8 atomes de carbone ou un groupe cycloalcényle de 5 à 8 atomes de carbone et

X représente l'azote ou un groupe CH,

caractérisé en ce qu'on fait réagir dans une première étape des racémates de dérivés azoliques de formule

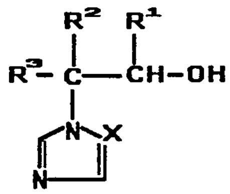

(Ia)

dans laquelle
$R^1$, $R^2$, $R^3$ et X ont la définition indiquée ci-dessus,
avec des halogénures optiquement actifs d'acide de perméthrine de formule

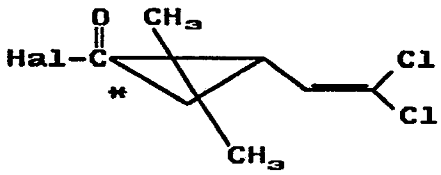

(II)

dans laquelle

Hal représente le chlore ou le brome, le cas échéant en présence d'un accepteur d'acide et en la présence éventuelle d'un diluant, puis dans une deuxième étape, on sépare les esters diastéréo-isomériques ainsi obtenus de formule

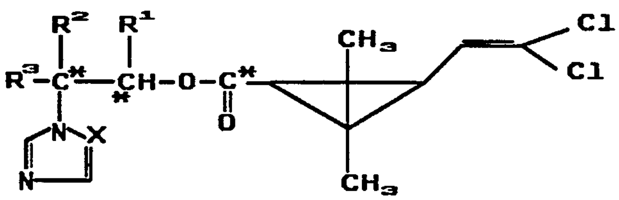

(III)

(mélange de diastéréo-isomères)

dans laquelle
$R^1$, $R^2$, $R^3$ et X ont la définition indiquée ci-dessus,
d'après les différences de leurs propriétés physiques, après quoi dans une troisième étape, on libère chaque dérivé azolique de formule (I) de l'ester correspondant à l'aide de bases en présence d'un diluant.

2. Procédé suivant la revendication 1, caractérisé en ce qu'on utilise, comme halogénure d'acide de perméthrine optiquement actif de formule (II), le chlorure d'acide de 1R-trans-perméthrine.

3. Procédé suivant la revendication 1, caractérisé en ce qu'on utilise, comme halogénure optiquement actif d'acide de perméthrine de formule (II), le chlorure d'acide 1S-trans-perméthrine.

4. Procédé suivant la revendication 1, caractérisé en ce qu'on conduit la première étape à des températures comprises entre -10°C et +150°C.

5. Procédé suivant la revendication 1, caractérisé en ce qu'on conduit la troisième étape à des températures comprises entre 0°C et 120°C.

6. Procédé suivant la revendication 1, caractérisé en ce qu'on utilise dans la conduite de la première étape, par mole de dérivé azolique racémique de formule (Ia), 1 à 2 moles d'halogénure d'acide de perméthrine optiquement actif de formule (II) et 1,5 à 2,5 moles d'accepteur d'acide.